# EUROPÄISCHE PATENTSCHRIFT

(11) **EP 3 393 544 B1**
(45) Veröffentlichungstag und Bekanntmachung des Hinweises auf die Patenterteilung: **27.01.2021**
(21) Anmeldenummer: 16822930.0
(22) Anmeldetag: 14.12.2016
(51) Int. Cl.: A61M 1/16, A61M 1/36, G16H 20/17, G16H 40/63

(54) **SENSORGESTEUERTE DISPLAYAUSGABE FÜR DIALYSEMASCHINEN**
SENSOR-CONTROLLED DISPLAY OUTPUT FOR DIALYSIS MACHINES
SORTIE D'AFFICHAGE COMMANDÉE PAR CAPTEUR POUR MACHINES DE DIALYSE

(30) Priorität: 21.12.2015 DE 102015122347
(43) Veröffentlichungstag der Anmeldung: 31.10.2018
(73) Patentinhaber: Fresenius Medical Care Deutschland GmbH, 61352 Bad Homburg (DE)
(72) Erfinder: DANIEL, Pia Nora, 78351 Bodman (DE)
(74) Vertreter: Schwarz, Claudia
(86) Internationale Anmeldenummer: PCT/EP2016/081000
(87) Internationale Veröffentlichungsnummer: WO 2017/108532

(56) Entgegenhaltungen:
- EP-A1- 0 623 357
- WO-A1-2004/103442
- WO-A1-2013/116660
- WO-A1-2014/162000
- WO-A2-2013/074769

## Beschreibung

Die vorliegende Erfindung betrifft eine Steuereinheit, eine Dialysemaschine oder ein anderes medizinisches Gerät und ein Verfahren sowie ein Computerprogramm zur sensorgesteuerten Displayansteuerung für eine Dialysemaschine.

Bekannte, moderne medizintechnische Geräte, insbesondere Dialysegeräte, beispielsweise das Hämodialysegerät 5008 von Fresenius Medical Care, erfordern zum Aufrüsten der Maschine oder zum Betrieb der Maschine eine Abfolge von Bedienschritten und Aktionen, die an der Maschine oder an angeschlossenen Betriebsmitteln, wie z.B. einer Heparinpumpe, ausgeführt werden müssen. Der Anwender wird bei den auszuführenden Bedienschritten über eine passende Darstellung auf einem Display der Maschine unterstützt und geleitet. Bei dem Display kann es sich z.B. um einen berührungssensitiven Bildschirm, einen Touchscreen, zur Steuerung des Gerätes und zur Ein- und Ausgabe von Daten handeln. Für die berührungssensitiven Bedienoberflächen der Dialysegeräte wird üblicherweise eine kapazitive Sensortechnik verwendet. Ein Touchscreen mit einer kapazitiven Sensortechnik ist beispielsweise in der DE10 2011 011 769 A1 oder der WO2004/103442 A1 näher beschrieben.

Das Hämodialysesystem umfasst als zentrale Einheit ein Dialysegerät, das dazu dient, Blut des Patienten kontinuierlich in ein extrakorporales technisches Kreislaufmodul durch eine Blutkammer eines Filters bzw. eines Dialysators zu leiten. Die Blutkammer ist über eine semipermeable Membran von einer Dialysierflüssigkeitskammer getrennt. Die Dialysierflüssigkeitskammer wird von einer Blutelektrolyte enthaltenden Dialysierflüssigkeit durchströmt. Die Stoffkonzentration der Dialysierflüssigkeit entspricht der Konzentration des Blutes eines Gesunden. Während der Behandlung werden das Blut des Patienten und die Dialysierflüssigkeit an beiden Seiten der Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Durch Anlegen eines Transmembrandrucks kann der Stoffwechsel zusätzlich beeinflusst werden.

Zur Ausführung der vorstehend beschriebenen Funktionalität umfasst das medizinische Gerät mit dem extrakorporalen Blutkreislaufmodul mehrere technische Bauteile, wie Substituatpumpen, Ventile, Drosseln, Drucksensoren sowie technische Elemente und Vorrichtungen, wie externe Anschlüsse, Schiebegriffe, Lüfterfilter, einen Stromanschluss, Hydraulikanschlüsse mit Klappen etc. Zum Aufrüsten und zum Betrieb des Dialysesystems müssen die vorstehend erwähnten technischen Bauteile und Vorrichtungen - häufig in einer aufeinander abgestimmten Reihenfolge mit mehreren Bedienschritten - bedient werden. Bei der Ausführung von Bedienschritten an den unterschiedlichen technischen Betriebsmitteln der Maschine kann es zu Fehlern oder Fehlbedingungen kommen, die den Betrieb des Dialysegerätes beeinträchtigen oder sogar unmöglich machen. Von daher ist es wichtig, eine korrekte Bedienung sicherzustellen. Fehler sollten grundsätzlich vermieden und möglichst umgehend erkannt werden. Dazu ist es im Stand der Technik bekannt, auf einem Display der Dialysemaschine bestimmte Hinweise auszugeben, mit denen der Anwender bei der Ausführung von Bedienschritten unterstützt wird. Dabei wird ein vorgegebenes festes Menü bereitgestellt und ausgegeben. Der Anwender erhält somit Hinweise über eine Abfolge von sequentiell auszuführenden Bedienschritten.

Als nachteilig erweist es sich jedoch bei den bekannten Systemen, dass die Darstellung auf dem Display durch ein vorgegebenes Menü mit einer festgelegten Menüstruktur unveränderlich definiert ist. Das Menü wird sozusagen in fixer Abfolge durchlaufen. In der Praxis hat es sich jedoch gezeigt, dass hier mehr Flexibilität gewünscht ist. Insbesondere sollte eine Displaydarstellung möglich sein, die an die aktuelle spezifische Situation angepasst ist, z.B. wenn der Anwender bestimmte Bedienschritte fehlerhaft ausgeführt hat, ist es wünschenswert, dass Displaydarstellungen mit Korrekturhinweisen ausgegeben werden, auch wenn sich der Anwender gerade in einem anderen Menü befindet. Dabei sollte eine korrekte Ausführung eines jeweiligen Bedienschrittes überprüft und abhängig vom Ergebnis der Überprüfung eine Ergebnis-basierte Displaydarstellung veranlasst werden, so dass z.B. bei der Detektion eines Fehlers, eine modifizierte Menüstruktur bereitgestellt und beispielsweise ein Hinweis ausgegeben kann, dass bei einem bestimmten Betriebsmittel ein Fehler erfasst worden ist - gegebenenfalls mit der Ausgabe von weiteren Korrekturmaßnahmen.

Da die Bedienung des Gerätes mitunter lebenserhaltende Maßnahmen für den Patienten betrifft, ist die Sicherstellung einer korrekten Bedienung des Gerätes mit allen Vorbereitungsschritten von hoher Bedeutung für die Sicherheit des Systems. In diesem Zusammenhang möchte die Erfindung die Mensch Maschine Schnittstelle verbessern.

Die vorliegende Erfindung hat sich deshalb zur Aufgabe gestellt, den Betrieb von Dialysegeräten und anderen medizinischen Geräten zu verbessern und insbesondere sicherer zu machen. Des Weiteren soll bei einem Gerätebetrieb mit mehreren unterschiedlichen Betriebsmitteln der Zustand aller Betriebsmittel, insbesondere auf Fehlerfreiheit, überwacht werden. Das Ergebnis der Überwachung sollte in eine verbesserte Displayansteuerung einfließen, mit der es möglich wird, die Darstellung auf dem Display in Abhängigkeit von einem Zustand des Gerätes und seiner Betriebsmittel auszulösen.

Diese Aufgabe wird erfindungsgemäß durch ein medizinisches Gerät und ein Verfahren sowie durch ein Computerprogrammprodukt gemäß den beiliegenden nebengeordneten Patentansprüchen gelöst.

Im Folgenden wird die Erfindung anhand der vorrichtungsgemäßen Aufgabenlösung und somit unter Bezugnahme auf die Steuerungseinheit beschrieben. Dabei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf ein Gerät gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch elektronische Schaltkreisbauteile oder Mikroprozessor-Module des Systems bzw. der Vorrichtung ausgebildet und umgekehrt.

Ein Aspekt betrifft eine Steuerungseinheit zur Ansteuerung eines Displays zum Betrieb eines medizinischen Gerätes, insbesondere eines Dialysegerätes. Dabei ist eine Sensoreinheit am medizinischen Gerät vorgesehen, die dazu bestimmt ist, physikalische Signale zu erfassen und an die Steuerungseinheit weiterzuleiten. Die Steuerungseinheit ist dazu bestimmt, aus den erfassten Signalen automatisch einen Zustand des medizinischen Gerätes zu ermitteln und mittels einer Steuerlogik Steuerbefehle zur zustandsabhängigen Ansteuerung des Displays zu berechnen und vorzugsweise auch auszugeben.

Im Kern betrifft die Steuerungseinheit ein elektronisches Modul für eine sensorbasierte und zustandsabhängige Ausgabe von Grafiksignalen und gegebenenfalls entsprechenden Steuerbefehlen zur Ansteuerung des bzw. zur Ausgabe auf dem Display/s. Je nach erfasstem Zustand sollen unterschiedliche zustandsspezifische Menüs zur Darstellung auf dem Display kommen, um den Anwender beim Betrieb des Gerätes zu unterstützen und/oder ihn auf etwaige Fehler und/oder nächste Schritte hinzuweisen.

Die bevorzugte Ausführungsform der Erfindung bezieht sich auf Betriebsmittel zur Aufnahme bzw. zum Einlegen von Einwegartikeln, wie beispielsweise einer Heparinspritze in eine Heparinpumpe einer Dialysemaschine und die dabei notwendige Bedienung des Betriebsmittels und deren Überprüfung auf Fehlerfreiheit über eine entsprechend spezifisch gestaltete Benutzerführung über das Display. Andere Ausführungsformen der Erfindung betreffen andere Betriebsmittel, wie z.B. andere Pumpen, Haltevorrichtungen - z.B. für einen Dialysefilter - und/oder Anschlüsse am Dialysegerät und/oder Aufnahmeeinrichtungen für medizinische Bauteile oder Einwegartikel.

Die Steuerungseinheit ist eine elektronische Einheit, die in Hardware als integrierter Schaltkreis (z.B. als FPGA, field-programmable gate array) oder in Software ausgebildet sein kann. Die Steuerungseinheit dient zur Displayansteuerung. Die Steuerungseinheit kann unmittelbar in einer Grafikkarte oder in einem Grafikchip implementiert sein oder mittelbar auf einer Prozessoreinheit, die mit der Grafikkarte und mit dem Display in Datenaustausch steht. Die Grafikkarte schreibt Daten für das Display auf einen Grafikspeicher, der üblicherweise als RAM (random access memory) ausgebildet ist. Die Prozessoreinheit und/oder der Grafikchip bzw. die Grafikkarte lesen den Speicher aus, um die gespeicherten Daten auf dem Display zur Anzeige zu bringen. Fakultativ kann ein Video-Adapter implementiert sein, der die digitale Signale der Steuerungseinheit und/oder eines Applikationsprogramms verwendet, diese im Speicher (z.B. Video RAM) speichert und ausgibt oder falls erforderlich in ein analoges Signal konvertiert (unter Verwendung eines D/A-Wandlers). Die Steuerungseinheit steht mit der Sensoreinheit in Datenaustausch. Dabei kann es sich vorzugsweise um eine unidirektionale Datenverbindung handeln, über die die Sensoreinheit erfassten Sensordaten an die Steuerungseinheit sendet. Die Sensordaten repräsentieren einen Zustand des medizinischen Gerätes mit vorzugsweise allen an das Gerät angeschlossenen Betriebsmitteln. In einfacheren Ausführungsform kann auch nur eine Auswahl der Betriebsmittel mit entsprechenden Sensoren ausgestattet sein, deren Zustand dann berücksichtigt wird. Die Sensordaten werden mittels einer Steuerlogik der Steuerungseinheit in Steuerbefehle zur zustandsabhängigen Ansteuerung des Displays zur Signalausgabe auf dem Display umgesetzt.

Die Steuerlogik ist ein integrierter Schaltkreis und/oder ein Programm, der/das bestimmt, wie die Sensordaten, die von unterschiedlichen Sensoren stammen können, verrechnet werden und welche Displayausgabe jeweils auf Basis der erfassten Daten erzeugt werden soll.

Die Sensoreinheit umfasst mehrere Sensormodule. Die Sensormodule umfassen ihrerseits mehrere Sensoren. Die Sensormodule sind an dem Dialysegerät, vorzugsweise an mehreren Positionen im Gerät, und an allen oder ausgewählten Betriebsmitteln des Gerätes und/oder an den jeweiligen Schnittstellen zwischen Gerät und Betriebsmittel verbaut. Die Sensoren sind vorzugsweise als Sensoren unterschiedlichen Sensortyps verbaut und umfassen neben optischen Sensoren, akustische Sensoren, Positions- und/oder Näherungssensoren, Temperatursensoren, Hallsensoren und anderen Sensortypen auch Schalter, Taster und/oder Potentiometer etc.

Das medizinische Gerät benötigt zum Betrieb eine Vielzahl von technischen Betriebsmitteln, wie Pumpen, Schläuche oder sonstige mechanische und/oder elektronische Einheiten, die an das Gerät angeschlossen werden müssen oder bereits in dieses integriert sind. Wie vorstehend bereits erwähnt, handelt es sich in einer bevorzugten Ausführungsform der Erfindung bei dem Betriebsmittel um eine Heparinpumpe. Andere Ausführungsformen der Erfindung beziehen sich auf andere Pumpen, Dialysefilter in Dialysatoren, Klemmen, Module oder Einrichtungen als Betriebsmittel. Das Betriebsmittel kann auch dazu bestimmt sein, Einwegartikel aufzunehmen, wie Schläuche, Filter, Einmalspritzen etc. In einer bevorzugten Ausführung der Erfindung ist jedes Betriebsmittel mit zumindest einem Sensor ausgebildet. Die Betriebsmittel müssen bedient werden. Zum Beispiel muss eine Heparinspritze korrekt in die dafür vorgesehene Pumpe am Gerät eingesetzt und angeschlossen werden, bevor dieses in Betrieb genommen werden kann. Es ist somit eine Abfolge von bestimmten Bedienschritten am Gerät und/oder an den Betriebsmitteln erforderlich. Diese Abfolge wird erfindungsgemäß durch die Sensoreinheit kontrolliert.

Die Gesamtheit aller Sensorsignale repräsentiert einen Zustand des Gerätes bzw. der Maschine mit seinen Betriebsmitteln. Der Zustand umfasst mehrere Zustandsgrößen, die abhängig von dem jeweiligen Anwendungsfall und den im Einsatz befindlichen Betriebsmitteln sind. So kann der Zustand unter anderem beispielsweise durch die Zustandsgröße "Türen oder Abdeckung am Dialysegerät 5008 geschlossen oder geöffnet?" definiert sein. Dies wird durch einen oder mehrere geeignete(n) Sensor(en) (z.B. Lagesensoren) erfasst. Ein Aufrüsten der Dialysemaschine mit dem Blutschlauchsystem kann z.B. erst dann stattfinden, wenn die Abdeckung geöffnet ist. Vor dem Öffnen der Abdeckung ist eine Darstellung von Menüpunkten, die spätere Aktionen zum Anschluss oder Betreiben des Blutschlauchsystems betreffen, nicht sinnvoll oder für den Anwender sogar verwirrend, da er sie ja in dem Zustand (noch) nicht ausführen kann. Erfindungsgemäß werden diese Menüpunkte in dem noch ungeöffneten Zustand auch nicht angezeigt, sondern es erfolgt eine Displayausgabe, die den Anwender z.B. auf die Notwendigkeit des Öffnens der Türen hinweist. In der Regel sind zum Aufrüsten oder zum Betrieb der Maschine mehrere Aktionen, Eingriffe (mit angeschlossenen technischen Vorrichtungen oder manuell betätigt), Prozessschritte oder Bedienschritte an unterschiedlichen Betriebsmitteln auszuführen. Dabei kann es notwendig sein, eine bestimmte Reihenfolge bei der Bedienung der einzelnen Betriebsmittel und/oder bei der Ausführung der einzelnen Bedienschritte einzuhalten. Erfindungsgemäß wird dazu abhängig von dem berechneten Zustand, der auf den erfassten Sensordaten basiert, eine spezifisch generierte Menüstruktur mit einer entsprechenden Abfolge von Displayausgaben erzeugt, die den Anwender im spezifischen Anwendungsfall zustandsabhängig unterstützt.

In einer bevorzugten Ausführungsform umfasst die Steuerungseinheit einen Schalter oder Taster zur Bestimmung eines Menütyps. Grundsätzlich sind zwei unterschiedliche Menütypen vorgesehen: ein Step-by-Step Modus, der eine Displaydarstellung für jeden Bedienschritt einer Folge von Bedienschritten erzeugt und diese sequentiell passend zur Ausführung der Bedienschritte am Gerät zur Anzeige bringt. Dabei kann ein Bedienschritt am Gerät oder an einem der Betriebsmittel einer Grafikausgabe mit einer entsprechenden Displaydarstellung zugeordnet sein. Die jeweilige Grafikausgabe kann wiederum mehrere untergeordnete Grafikausgaben (z.B. in Form von Menü-Unterseiten) umfassen. Dies hat zur Folge, dass der Anwender durch die Darstellung der Abfolge von Menüseiten bei der Abfolge von Bedienschritten geführt wird. Des Weiteren ist ein parameter-basierter Modus vorgesehen, bei dem Maschinenparameter direkt eingestellt werden können. Dies hat den Vorteil, dass ein versierter Anwender sich nicht durch das vollständige Menü klicken muss, sondern die jeweiligen Einstellungen direkt eingeben kann. Der Schalter oder Taster kann auch als sogenannter touch key auf dem Display ausgebildet sein. Des Weiteren kann anstelle des Schalters oder Tasters eine automatisch oder halbautomatisch betriebene Bestimmungseinheit vorgesehen sein, die dazu bestimmt ist, den Menütyp zu erfassen oder bestimmen.

Das Display stellt eine Benutzeroberfläche (auch Monitor genannt) dar. Es ist vorzugsweise als berührungssensitives Display ausgebildet und umfasst einen Sensor (Touchscreensensor) zur Erfassung der Eingabesignale (z.B. auf Eingabefeldern) zur Steuerung und Bedienung des Dialysegerätes (im Folgenden auch Gerät oder Maschine genannt) mit seinen Betriebsmitteln sowie ein Display, auf dem Interaktionsflächen, Schaltelemente, Kontrollfelder etc. zur Steuerung des Dialysegerätes dargestellt sind. Das Display dient insbesondere zur Darstellung eines spezifisch erzeugten Menüs mit einer Abfolge von unterschiedlichen Menüseiten zur Steuerung oder zum Betrieb des medizintechnischen Gerätes. Das Menü wird vor oder während der Ausführung von Bedienschritten am Gerät angezeigt und dient dazu, den Anwender bei der Ausführung der Bedienschritte zu führen oder ihm Zusatzinformationen zur Verfügung zu stellen. Bei dem Display handelt es sich somit um eine Ein- und Ausgabeeinheit bzw. Benutzer-Schnittstelle zur Ein- und Ausgabe von Signalen. Das Display ist vorzugsweise als kapazitiver Touchscreen ausgebildet. Vorzugsweise verfügt der Touchscreen über eine Multi-Sensor-Funktionalität, so dass auch gleichzeitige Berührungen detektiert werden können. Ein Beispiel eines solchen Touchscreens ist in der DE10 2011 011 769 A1 näher beschrieben. Üblicherweise umfasst der Touchscreen neben dem Display (der eigentlichen Anzeigeeinheit) einen Touchscreensensor als Eingabeeinheit für Anwendersignale, einen Controller und fakultativ einen Treiber, der in dem medizinischen Gerät angeordnet sein kann. In einer alternativen und ebenfalls bevorzugten Ausführungsform der Erfindung kann der Touchscreensensor als projiziert-kapazitiver Sensor ausgebildet sein (meistens "PCT" = "Projected Capacitive Touch" oder "PCAP" genannt). Dabei nutzt der Sensor zwei Ebenen mit einem leitfähigen Muster (beispielsweise Streifen oder Rauten). Die Ebenen sind voneinander isoliert angebracht. Befindet sich ein Finger am Kreuzungspunkt zweier Streifen, so ändert sich die Kapazität des Kondensators, und es kommt ein größeres Signal am Empfängerstreifen an. Diese Signaländerung lässt sich somit genau anhand der X-und Y-Koordinaten messen, wobei auch mehrere Berührungspunkte exakt definierbar sind. Der Stromfluss von den Ecken des Touchscreens zum Berührungspunkt ist proportional zu den XY-Koordinaten. Der wesentliche Vorteil dieses Systems ist, dass der Sensor auf der Rückseite des Deckglases angebracht werden kann, da die Berührungserkennung durch das Glas "hindurchprojiziert" wird. So erfolgt die Bedienung auf der praktisch verschleißfreien Glasoberfläche. Ferner ist die Erkennung von Gesten und mehreren Berührungen (also Multi-Touch) möglich. In anderen Ausführungsform der Erfindung können jedoch auch resistive oder induktive oder andere Sensortechnologien für Touchscreens zur Anwendung kommen.

Im Folgenden wird die Erfindung für ein Dialysegerät als Beispiel eines medizinischen Gerätes beschrieben, z.B. eines Hämodialysegerätes oder eines Peritonealdialysegerätes. Für den Fachmann liegt es jedoch auf der Hand, dass die Erfindung ebenso auf andere medizintechnische, computergesteuerte Geräte oder (Fluidmanagement-) Maschinen oder Blutbehandlungsgeräte angewendet oder übertragen werden kann, die über ein Display zur Anzeige von Gerätedaten verfügen und/oder die über das Display gesteuert werden.

In einer bevorzugten Ausführungsform der Erfindung ist der Zustand mitunter durch den aktuellen oder durch den geplanten Betrieb des Gerätes definiert. Der aktuelle Zustand wird durch die Sensoreinheit erfasst und betrifft somit einen IST-Zustand des Systems mit all seinen Betriebsmitteln. In einer Weiterbildung kann aus dem erfassten IST-Zustand auch ein prognostizierter wahrscheinlicher Folge-Zustand berechnet werden. So kann z.B. durch geeignete Sensoren erfasst werden, ob ein Single-Needle- oder ein Zweinadelbetrieb oder ein Betrieb mit einer Substituatpumpe ausgeführt werden soll. Nach Erfassen des Zustandes werden dann erfindungsgemäß automatisch zustandsabhängige Ausgabesignale zur Anzeige auf dem Display erzeugt. Nachdem z.B. durch die Sensoreinheit automatisch erfasst wurde, dass die Single-Needle-Pumpe bestückt werden soll, erkennt die erfindungsgemäße Steuerungseinheit automatisch, dass ein Single-Needle-Betrieb ausgeführt werden soll (Folge-Zustand) und erzeugt Ausgabesignale und entsprechende Ansteuerbefehle, um ein Menü zur Aufrüstung der Maschine im Single-Needle- Betrieb anzuzeigen.

In einer bevorzugten Ausführungsform der Erfindung ist die Sensoreinheit auf allen Betriebsmitteln angeordnet. In einfacheren Ausführungsformen sind Sensoren der Sensoreinheit nur auf relevanten und ausgewählten Betriebsmitteln ausgebildet. Die Sensoreinheit umfasst zumindest einen und vorzugsweise mehrere Sensoren an unterschiedlichen Bauteilen und/oder Positionen und/oder Betriebsmitteln des medizinischen Gerätes. Die Sensoren sind dazu ausgebildet, unterschiedliche technische Signale, insbesondere physikalische, elektrische und/oder chemische Messgrößen, zu erfassen. Die Sensoren können als optischer Sensor (z.B. in Form einer Kamera), als akustischer Sensor, Hallsensor, Positionssensor und/oder Sensor zur Erfassung anderer Signale ausgebildet sein. Die Sensoreinheit kann zumindest einen der vorstehend erwähnten Sensoren, einen Schalter, Taster und/oder einen Potentiometer umfassen.

Die Steuerungseinheit weist ein einer bevorzugten Weiterbildung der Erfindung einen Sensortrigger-Schalter auf, über den sie aktivierbar und deaktivierbar ist. Die Funktion der Sensoreinheit kann somit vorteilhafterweise zu oder abgeschaltet werden.

Gemäß einem anderen Aspekt wird die Aufgabe gelöst durch ein medizinisches Gerät, insbesondere ein Dialysegerät, das über ein Display bedient und gesteuert wird, mit folgenden in Datenaustausch stehenden Modulen:
- Einer Steuerungseinheit, wie vorstehend beschrieben;
- Einer Mehrzahl von technischen Betriebsmitteln, die an das medizinische Gerät angeschlossen sind oder zum Anschluss bestimmt sind (und noch angeschlossen werden müssen) und zum Aufrüsten oder Betrieb des medizinischen Gerätes bedient werden müssen;
- Einem Display, auf dem zur Bedienung des medizinischen Gerätes und/oder seiner Betriebsmittel Displayausgaben aus einem Grafikspeicher ausgegeben werden. Bei den Displayausgaben kann es sich um zustandsabhängig erzeugte Menüdarstellungen mit Untermenüseiten handeln.
- Einer Sensoreinheit, die am medizinischen Gerät und insbesondere an dessen Betriebsmitteln angeordnet ist und die dazu bestimmt ist, automatisch einen Zustand des medizinischen Gerätes mit allen oder ausgewählten Betriebsmitteln zu erfassen und zur Berechnung der Steuerbefehle an die Steuereinheit weiterzuleiten.

Gemäß einem anderen Aspekt wird die Aufgabe gelöst durch ein Verfahren zur Ansteuerung eines Displays eines medizinischen Gerätes, insbesondere eines Dialysegerätes, das über das Display bedient und gesteuert wird, mit folgenden Verfahrensschritten:
- Erfassen von Sensordaten, die einen Zustand des medizinischen Gerätes mit seinen Betriebsmitteln repräsentieren
- Berechnen von Steuerbefehlen zur zustandsabhängigen Ansteuerung des Displays in Abhängigkeit von den erfassten Sensordaten;
- Darstellen von unterschiedlichen zustandsspezifischen Menüs je nach erfasstem Zustand, um den Anwender beim Betrieb des Gerätes zu unterstützen und/oder ihn auf etwaige Fehler und/ oder nächste Schritte hinzuweisen, wobei eine Ausgabe auf dem Display (D) für genau dasjenige Betriebsmittel (200) ausgegeben wird, auf dem eine Zustandveränderung erfasst worden ist; und
- Speichern der Displayausgabe und einem der Displayausgabe zugeordnetem Zustand in einem Grafikspeicher (1003).

In einer bevorzugten Ausführungsform des Verfahrens erfolgt weiterhin auch ein Anwenden der Steuerbefehle zur Ausgabe einer Displaydarstellung in Abhängigkeit von den erfassten Sensordaten. Dies hat den Vorteil, dass der Anwender über die spezifisch und zustandsabhängig erzeugte Grafikausgabe bei der Bedienung des Gerätes unterstützt wird. In einer weiteren, bevorzugten Ausführungsform des Verfahrens umfasst der Zustand zumindest eine Zustandsgröße. Vorzugsweise charakterisiert jeweils eine Zustandsgröße einen Zustand jeweils eines Betriebsmittels. Insbesondere umfasst der Zustand somit alle Zustandsgrößen bzw. Zustände von allen relevanten Betriebsmitteln. Die Zustandsgröße kann nur einen Parameter haben und somit als binäres Signal ("1" oder "0") ausgebildet sein (z.B. repräsentierend für "Tür auf" oder "Türe zu"). Alternativ kann die Zustandsgröße auch mehrere Parameter umfassen (z.B. als Abstandssignal).

In der Regel erfordert die Bedienung eines jeweiligen Betriebsmittels eine Abfolge von Bedienschritten, die am Gerät oder an den Betriebsmitteln auszuführen sind. Im Step-by-Step Modus ist dabei jeweils ein Bedienschritt zumindest einer Displayausgabe zugeordnet ist. Dies hat den Vorteil, dass der Anwender für jeden Bedienschritt eine Anleitung oder einen Hinweis erhält. Vorzugsweise erfolgt die Ausgabe der erzeugten Grafik mit dem jeweiligen Menü in zeitlicher Hinsicht vor der Ausführung des Bedienschrittes und damit als Hinweis und Anleitung. Ebenso ist es möglich, die erzeugte Signalausgabe mit dem darzustellenden Menü erst nach der Ausführung des Bedienschrittes auszuführen und damit als Bestätigung für einen korrekten oder einen fehlerhaften oder unterlassenen Bedienschritt als Korrektur- und Überprüfungsausgabe.

Die Ausgabe auf dem Display kann eine interaktive Benutzerführung mit einer Abfolge von vorzugsweise hierarchisch strukturierten Betriebsmenüdarstellungen mit Untermenüdarstellungen für jeweils ein Betriebsmittel sein. Die Abfolge von Betriebsmenüdarstellungen wird in Abhängigkeit von den erfassten Sensordaten (und damit abhängig vom Zustand) bestimmt. Erfindungsgemäß wird auch während der Darstellung eines Menüs auf dem Display eine geänderte Displayausgabe erzeugt und ausgeben, wenn mittels der Sensoreinheit ein bestimmter Zustand erfasst wird, der eine Ausgabe eines Hinweises notwendig macht. Diese geänderte Displayausgabe wird auch dann angezeigt, wenn sich der Zustand auf ein anderes Betriebsmittel bezieht. Wenn also beispielsweise das Menü zum Anschließen der Substituatpumpe angezeigt wird und gleichzeitig aber erfasst wird, dass ein Fehler beim Einlegen der Heparinspritze in die Heparinpumpe erfolgt, dann wird das bisherige Menü sozusagen unterbrochen und es wird eine Bildseite ausgegeben, die Korrekturhinweise zur Fehlerbehebung beim Einlegen der Heparinspritze umfasst. Erfindungsgemäß wird es konfiguriert, dass eine Grafikausgabe für genau dasjenige Betriebsmittel ausgegeben wird, auf dem eine Zustandsveränderung erfasst worden ist. Dies hat den Vorteil, dass nur aktuelle und relevante Grafikausgaben ausgegeben werden und insbesondere solche, die sich auf Betriebsmittel beziehen, die aktuell gerade bedient worden sind. Weiterhin kann es konfiguriert werden, wann und wie die zustandsabhängigen Grafikausgaben ausgegeben werden. So kann eingestellt werden, dass die erfindungsgemäß erzeugte geänderte Grafikausgabe als zusätzliches Fenster über die bisherige Displayanzeige eingeblendet wird (als Pop-Up oder Float-In Fenster) und/oder dass die Ausgabe direkt und ohne Zeitverzug ausgegeben werden soll oder später mit einer Zeitverzögerung, z.B. erst nach Durchlauf durch ein aktuell auf dem Display ausgegebenes Menü (um den Anwender vorteilhafterweise nicht durch unterschiedliche Anweisungen zu verwirren).

In einer weiteren, bevorzugten Ausführungsform erfolgt die Ausgabe auf dem Display zur interaktiven Benutzerführung während des Betriebs oder während eines Aufrüstens des medizinischen Gerätes. Abhängig von den erfassten Sensordaten wird eine Betriebsmenüdarstellung für das jeweilige Betriebsmittel (auf dem die Sensordaten erfasst worden sind) erzeugt oder ist bereits vorkonfiguriert und wird zur Anzeige ausgewählt.

Eine weitere Aufgabenlösung besteht in einem Computerprogrammprodukt für ein medizinisches Gerät, das in einen Speicher eines Computers oder eines elektronischen oder medizintechnischen Gerätes geladen oder ladbar ist mit einem Computerprogramm zur Durchführung des oben näher beschriebenen Verfahrens, wenn das Computerprogramm auf dem Computer oder dem elektronischen oder medizintechnischen Gerät ausgeführt wird. Das Computerprogrammprodukt kann in einen internen Speicher einer digitalen Steuerungseinheit geladen werden.

Ein Computerprogramm zur Durchführung aller Verfahrensschritte des oben näher beschriebenen Verfahrens wird auch beschrieben, wenn das Computerprogramm auf einem Computer, einem elektronischen oder medizintechnischen Gerät ausgeführt wird. Dabei ist es auch möglich, dass das Computerprogramm auf einem für den Computer oder das elektronische oder medizintechnische Gerät lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

- Fig. 1: zeigt ein Dialysegerät mit einem Display gemäß einer bevorzugten Ausführungsform der Erfindung in einer Prinzipdarstellung.
- Fig. 2: ist eine schematische Darstellung eines Dialysegerätes mit einer erfindungsgemäßen Steuerungseinheit und einem medizinischen Gerät.
- Fig. 3: ist eine detaillierte Darstellung einer Heparinpumpe, wobei
- Fig. 3a: eine Heparinpumpe ohne eingelegte Heparinspritze und
- Fig. 3b: eine Heparinpumpe mit eingelegter Heparinspritze darstellt.
- Fig. 4: zeigt ein Ablaufdiagramm gemäß einer bevorzugten Ausführungsform.
- Fig. 5: stellt eine beispielhafte schematische Darstellung für einen modifizierten Bildschirmaufbau mit einer zustandsspezifischen Displayausgabe dar.

### Detaillierte Beschreibung der Figuren

Im Folgenden wird die Erfindung anhand von Ausführungsbeispielen im Zusammenhang mit den Figuren näher beschrieben.

Die Erfindung verbessert die Mensch-Maschine-Schnittstelle hinsichtlich der Aufrüstzeit, der Bedienungszeit und hinsichtlich der Fehlerfreiheit. Durch die zustandsspezifische Erzeugung einer Displayausgabe auf dem Display D mit einer detaillierten Anzeige von erforderlichen Bedienschritten können Anwendungsfehler vermieden werden. Dies betrifft zunächst alle Bedieneingriffe am medizinischen Gerät 20 und seinen Betriebsmitteln 200 und insbesondere bei solchen Betriebsmitteln, die zum Einlegen von Einwegartikeln bestimmt sind, wie z.B. Pumpen oder Kupplungen für Schläuche etc.

Die Erfindung betrifft insbesondere eine automatische Detektion von Eingriffen an dem medizinischen Gerät 20 und dessen Betriebsmitteln 200 über eine Sensoreinheit S zur zustandsabhängigen Displayausgabe im Rahmen einer Benutzerführung bei der Gerätesteuerung.

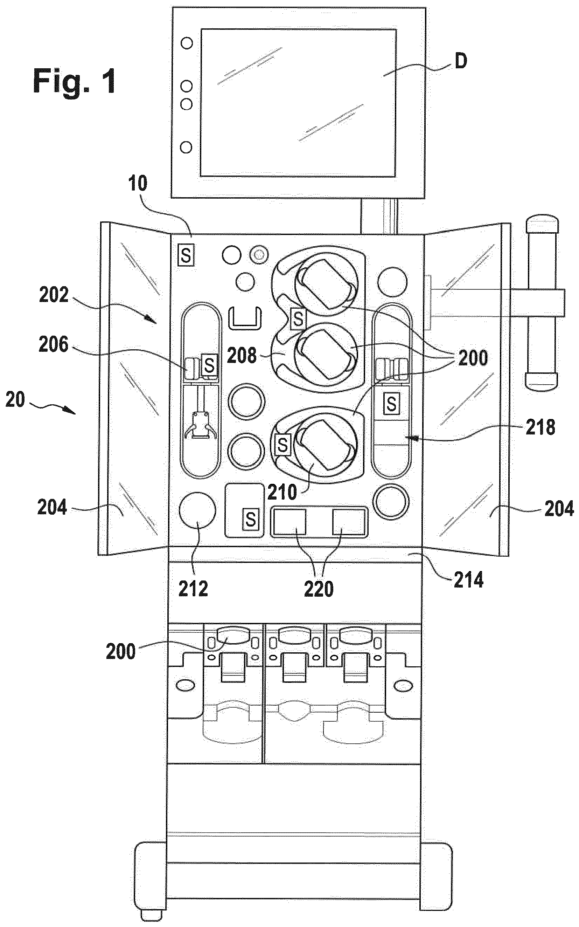

**Figur** 1 zeigt ein Dialysegerät als Vertreter eines medizinischen Gerätes 20 in einer Prinzipdarstellung. Das Dialysegerät wird über ein angeschlossenes Display D bedient und gesteuert. Erfindungsgemäß ist an das medizinische Gerät 20 eine Steuerungseinheit 10 über eine geeignete Datenverbindung angeschlossen. Das Dialysegerät umfasst, wie in Figur 1 schematisch gezeigt, als zentrales Element ein extrakorporales Behandlungsmodul 202. Dieses ist mit einer transparenten Abdeckung 204, hier in Form einer zweiflügeligen Türe vor ungewollten Berührungen gesichert. Das extrakorporale Behandlungsmodul 202 umfasst eine Vielzahl von Betriebsmitteln 200 in Form von unterschiedlichen Pumpen (z.B. Blut, Substituat), Ventilen, Spritzen, Halterungen, Aufnahmen und dergleichen. Auch das medizinische Gerät 20 umfasst Betriebsmittel 200. Die Betriebsmittel 200 können sich an unterschiedlichen Positionen entweder innerhalb des medizinischen Gerätes 20 oder außerhalb (an einer angeschlossenen Baueinheit) befinden, also auch außerhalb des extrakorporalen Behandlungsmoduls 202. Darüber hinaus können die Betriebsmittel 200 selbst auch als externe, separate Vorrichtung über eine entsprechende Verbindung und eine Datenverbindung an das Dialysegerät angeschlossen sein. In dem in Figur 1 gezeigten Ausführungsbeispiel ist im oberen mittigen Bereich des extrakorporalen Behandlungsmoduls 202 eine Blutpumpe 208 dargestellt. Darunter befindet sich eine Substituatpumpe 210 und auf der linken Seite unter einer Heparinpumpe 206 ein Substituatport 212. Am unteren Teil des extrakorporalen Behandlungsmoduls 202 befindet sich eine Rinne 214 mit einem Leckagesensor für das extrakorporale Behandlungsmodul 202 sowie unterschiedliche Messköpfe 220. Auf der rechten Seite ist eine Bauteilgruppe 218 zur venösen Füllstands- und Luftüberwachung ausgebildet, die z.B. einen optischen Detektor und einen Luftblasendetektor, eine Spannhebel mit Füllstandsdetektor (mittig dargestellt) mit einer Schlauchaufnahme bzw. Schlauchführung (im oberen Bereich) und einer Fixierung für einen venösen Blasenfänger umfassen kann. Des Weiteren können weitere Messeinheiten, Anschlüsse und/oder weitere Bauteile in dem extrakorporalen Behandlungsmodul 202 ausgebildet sein.

Erfindungsgemäß ist eine Sensoreinheit S vorgesehen, die eine Vielzahl von einzelnen Signalaufnehmern oder Sensoren umfasst. Die separaten Sensoren können von einem unterschiedlichen Typ sein oder dieselben physikalischen Größen erfassen. Es können z.B. optische Sensoren, Positionssensoren, Endlagesensoren, Hallsensoren oder Sensoren von einem anderen Typ vorgesehen sein. Die Sensoren sind entweder unmittelbar an dem Dialysegerät oder mittelbar an den Betriebsmitteln 200 des Dialysegerätes integriert oder befestigt. Die Betriebsmittel 200 dienen zum Betrieb oder zur Steuerung des medizinischen Gerätes 20. Die Betriebsmittel 200 können auch ausgelegt sein, um mit weiteren Bauteilen verbunden zu werden (z.B. Anschlüsse, Schläuche etc.). Darüber hinaus können die Betriebsmittel auch zur Aufnahme von Einwegartikeln (z.B. Spritzen, Schläuchen) dienen.

In einer bevorzugten Ausführung bezieht sich die Erfindung auf eine Heparinpumpe 206 als Betriebsmittel, die zum Eingriff mit einer Heparinspritze (in Figur 1 nicht dargestellt) bestimmt ist.

Wie vorstehend kurz erwähnt, können sich die Betriebsmittel 200 auch im Inneren des medizinischen Gerätes 20 und insbesondere im Inneren des extrakorporalen Blutbehandlungsmoduls befinden. Dabei kann es sich z.B. um Filter oder Pumpen handeln. Diese Betriebsmittel 200 müssen gewartet und/oder im Fehlerfall repariert werden. Dazu muss das extrakorporale Blutbehandlungsmodul (z.B. durch Abklappen einer Vorderseite) geöffnet werden. Der Zustand des Geöffnetseins kann dabei ebenfalls von der erfindungsgemäßen Sensoreinheit S detektiert und zur Berechnung in die Steuerungseinheit 10 weitergeleitet werden. In einer Weiterbildung kann die Steuerungseinheit 10 dazu ausgebildet sein, diese Sensordaten mit Wartungsplanungsdaten abzugleichen, die vorzugweise über eine bereitgestellte weitere (vorzugsweise netzwerkbasierte) Schnittstelle von einem zentralen Server oder einer Monitoringeinheit eingelesen werden. Falls der Abgleich ergibt, dass das Gerät 20 geöffnet ist (Sensorsignal) und in diesem Zeitraum ein Wartungsvorgang geplant ist (Serverdaten), dann kann erfindungsgemäß darauf geschlossen werden, dass eine Wartung als nächster Betriebseingriff zu erwarten ist. Dementsprechend wird ein jeweils zugeordnetes Menü (hier: zur Wartung des jeweiligen Betriebsmittels, z.B. als Menüstart-Bildseite 500) auf dem Display D ausgegeben.

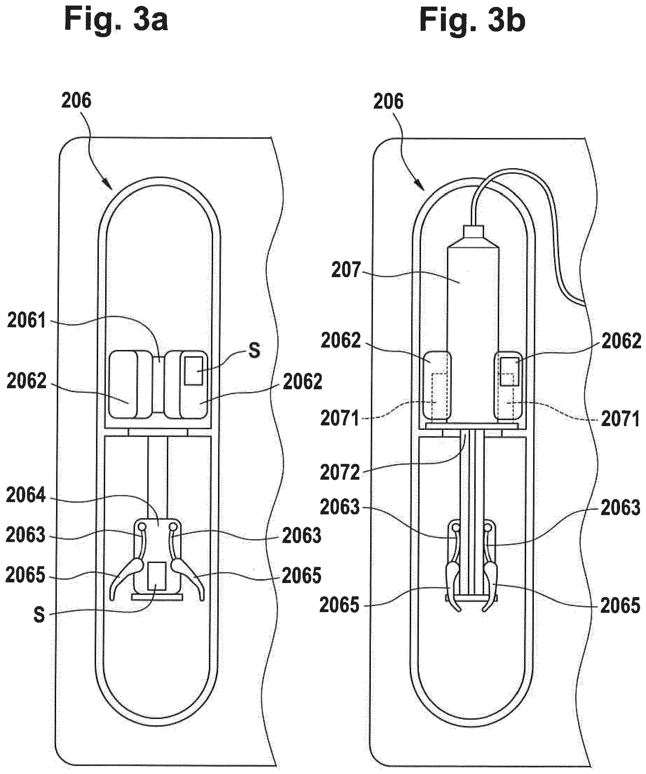

Die in Figur 1 dargestellte Heparinpumpe 206 wird in **Figur 3** nochmals detaillierter gezeigt und beschrieben. **Figur 3a** zeigt die Heparinpumpe ohne eingelegte Heparinspritze und **Figur 3b** mit eingelegter Heparinspritze 207. Für den Betrieb des Dialysegerätes muss die Heparinspritze 207 für die jeweilige Anwendung korrekt in die Heparinpumpe 206 eingelegt und dort arretiert werden. Die Heparinpumpe 206 umfasst dazu einen Spannhebel 2061 mit Spritzenerkennung, Haltebügel 2062, Klammern 2063, ein Griffstück 2064 und einen Klemmhebel 2065.

Die vorstehend erwähnten Betriebsmittel müssen korrekt bedient werden. Erfindungsgemäß kann dabei z.B. - je nach erfasstem Zustand - folgender Warnhinweis auf dem Display D ausgegeben werden:
- "Damit automatisch geprüft werden kann, ob die Heparinspritze korrekt fixiert und angeschlossen ist, sollte diese vor Erreichen des Spülvolumens eingelegt werden."
- "Es dürfen nur Heparinspritzen mit einem Volumen von kleiner oder gleich 30 ml verwendet werden."

Das Display D, auch Monitor genannt, dient als Mensch-Maschine Schnittstelle zur Ein- und Ausgabe von Signalen und Hinweisen zur Bedienung des medizinischen Gerätes 20 mit seinen Betriebsmitteln 200.

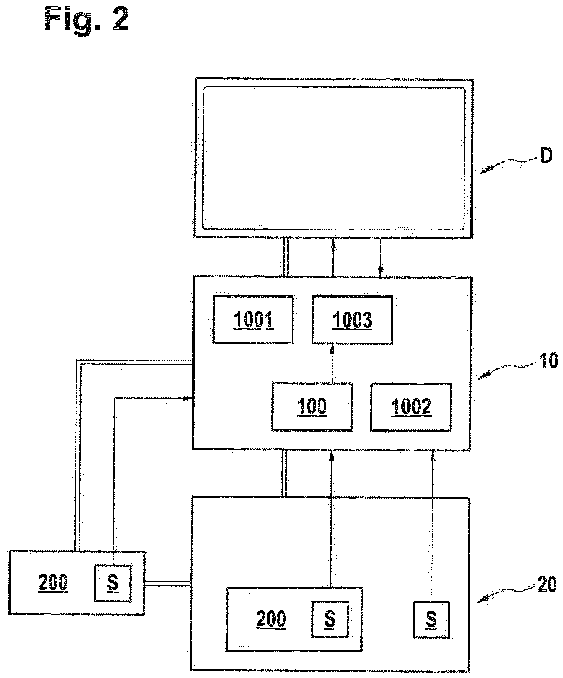

**Figur 2** beschreibt schematisch weitere Bauteile des medizinischen Gerätes 20 und der Steuerungseinheit 10. Ein Treiber 1001 kann in der Steuerungseinheit 10 oder in dem medizinischen Gerät 20 angeordnet sein, während ein Controller 1002, der als Microcontroller ausgebildet sein kann, direkt in dem Display D oder auch in der Steuerungseinheit 10 angeordnet ist oder die Verbindung dazu darstellt und sozusagen als Schnittstelle ausgebildet ist. Alternativ kann der Controller 1002 auch als separate Instanz ausgebildet sein, die über eine Datenverbindung angeschlossen ist. Die Steuerungseinheit 10 umfasst einen logischen Schaltkreis bzw. eine Steuerlogik 100. Die Steuerlogik 100 dient dazu, aus den von der Sensoreinheit S empfangenen Signalen einen Zustand des medizinischen Gerätes 20 mit all seinen Betriebsmitteln 200 zu errechnen und in Abhängigkeit von dem berechneten Zustand Steuerbefehle zu erzeugen, um eine fallspezifischen Displayausgabe zu erzeugen und in einen Grafikspeicher 1003 zu speichern und diese dann zur Darstellung auf den Display D auszulesen. Wie in Figur 2 angedeutet, sind normalerweise verschiedene Instanzen der Sensoreinheit ausgebildet, d.h. unterschiedlichen Sensoren, die an unterschiedlichen Bauteilen und Positionen am Gerät 20 angeordnet oder integriert sind und deren Signale an die Steuerungseinheit 10 zu automatischen Verrechnung übertragen werden. Die Sensoren können direkt an dem medizinischen Gerät 20 integriert sein und/oder indirekt an Betriebsmitteln 200 des Gerätes 20. Dabei können die Betriebsmittel 200 sich auch außerhalb des Gerätes 20 befinden und mit diesem in Datenaustausch stehen und/oder auf sonstige Weise verbunden sein.

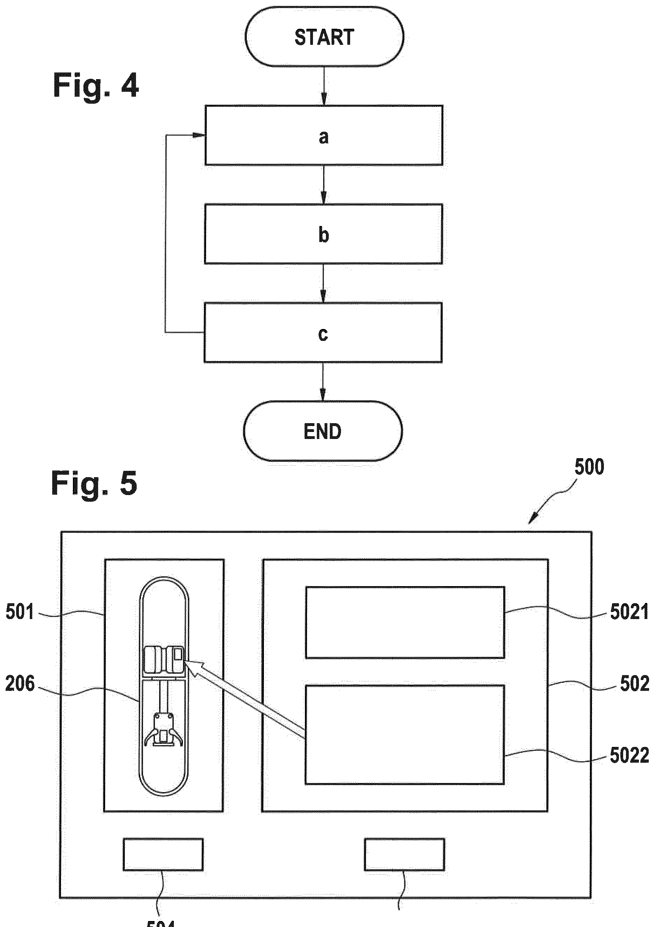

**Figur 4** zeigt ein Ablaufdiagramm gemäß einer bevorzugten Ausführung der Erfindung. Nach dem Start des Systems erfolgt in **schritt a** das Erfassen von Sensordaten Dabei können - je nach Konfiguration des Verfahrens - alle Sensoren der Sensoreinheit S abgefragt werden oder nur eine vordefinierte Auswahl der Sensoren. Letzteres hat den Vorteil, dass die zu übertragende und zu verrechnende Datenmenge nicht zu groß wird und die Zeitdauer des Verfahrens insgesamt damit möglichst kurz gehalten werden kann. Die erfassten Sensorsignale werden der Steuerungseinheit 10 und der Steuerlogik 100 zugeführt, um daraus einen Zustand des Gerätes 20 mit allen relevanten Betriebsmitteln 200 zu berechnen. Der Zustand kennzeichnet, an welchem Betriebsmittel 200 und/oder an welcher Position oder an welchem Bauteil des Gerätes 20 ein Benutzereingriff detektiert worden ist. Wird z.B. ein Einmalartikel in die Nähe des Gerätes 20 gebracht, wird dies automatisch mit geeigneten Sensoren (optische Sensoren und/oder Näherungssensoren) erfasst. Als Sensoren werden vorzugsweise passiv betriebene Transponder (also ohne eigene Stromversorgung) verwendet, die ihre erfassten Sensorsignale und Daten drahtlos (z.B. mittels Radiowellen) an einen aktiv betriebenen Empfänger (Lesegerät) übertragen, wie z.B. um RFID-Tags und/oder NFC-Sensoren. In Abhängigkeit von dem berechneten Zustand oder von den erfassten Sensorsignalen wird in **Schritt b** eine jeweils aktuelle und spezifische grafische und/oder textuelle Ausgabe zur Darstellung auf dem Display D erzeugt. Die Ausgabe wird **in Schritt c** auf dem Display D ausgegeben und kann beispielsweise zumindest eine neu erzeugte Bildseite 500 umfassen mit einer zwei- oder dreidimensionalen Darstellung des erfassten Einwegartikels und zusätzlichen grafischen Ausgaben für den Einwegartikel, z.B. mit Hinweisen, was beim Einlegen des Artikels in das jeweilige Betriebsmittel 200 oder in das Gerät 20 zu beachten ist. Weiterhin können mitunter Markierungen auf dem Display zur Anzeige gebracht werden, um den Anwender bei der Bedienung zu unterstützen. Dabei weisen die Markierungen auf Positionen am Gerät 20 und/oder auf Positionen an den Betriebsmitteln 200 hin, die jeweils als Nächstes bedient werden müssen. Somit umfasst die zustandsabhängige Ansteuerung des Displays D auch einen Zeitaspekt. Damit wird der Anwender zum einen hinsichtlich der zu bedienenden Bauteile geführt und zum anderen, wann diese Bauteile bzw. Betriebsmittel 200 und/oder in welcher Reihenfolge bedient werden müssen. Nachdem eine Displayausgabe erzeugt und auf dem Display D zur Anzeige gebracht worden ist, kann die weitere Bedienung des Gerätes 20 wieder über die Sensoreinheit S überwacht werden. Dies ist in Figur 4 durch den Pfeil von Schritt c zu Schritt a repräsentiert. Andernfalls endet das Verfahren oder wird erneut durchlaufen. Wichtig ist, dass die Ausgabe einer geänderten Bildseite als zustandsabhängige Displayausgabe immer dann erfolgt, wenn eine Zustandsänderung an einem Betriebsmittel 200 erfasst worden ist.

**Figur** 5 zeigt exemplarisch eine solche Bildschirmausgabe in einem Anwendungsfall für eine Heparinpumpe 206. Nachdem die Sensoreinheit S einen Eingriff an der Heparinpumpe 206 erkannt hat und erfasst hat, dass noch keine Heparinspritze 207 eingelegt worden ist, wird automatisch eine zustandsspezifische Ausgabe 500 erzeugt, die den Anwender bei seinen nächsten Schritten führt. Der Anwender legt daraufhin eine Spritze in die Spritzenhalterung ein. Dies wird erfindungsgemäß über Sensoren in den Flügelhalterungen erkannt. Unabhängig davon, welcher Inhalt gerade auf dem Display D angezeigt wird, wird die Displayausgabe nun erfindungsgemäß modifiziert. Es wird insbesondere eine neue Bildseite 500 ausgegeben, in der eine gezoomte dreidimensionale Darstellung 501 der Spritzenhalterung (in Figur 5 auf der linken Seite) und eine zustandsspezifisches Hinweisfeld 502 (in Figur 5 auf der rechten Seite) angezeigt wird. Die Größe, Position und/oder die Art der Felder können erfindungsgemäß konfiguriert werden. Die dreidimensionale Darstellung 501 bezieht sich auf ein technisches Bauteil (hier: Heparinspritze 207), das zum Eingriff mit einem Betriebsmittel 200 (hier: Heparinpumpe 206) bestimmt ist. Das Hinweisfeld 502 kann zumindest ein Textfeld 5021 und/oder zumindest ein Markierfeld 5022 umfassen. Das Markierfeld dient zur Ausgabe von Hinweisen (z.B. in Form von Pfeilen, die auf Bedienelemente verweisen, die in der Bilddarstellung 501 dargestellt sind) auf die entsprechenden Positionen in der Bilddarstellung 501, die als jeweils Nächstes zu bedienen sind. Vorzugsweise entspricht der Inhalt des Textfeldes 5021 dem im Markierfeld 5022 dargestellten Hinweis. Der Hinweis kann auch in Form einer Hervorhebung oder Markierungen in der Bilddarstellung 501 ausgebildet sein. Die Bildseite 500 kann zusätzlich noch Bestätigungsfelder 504 umfassen, die dazu dienen, ein benutzerseitig eingegebenes Bestätigungssignal zu erfassen. Dieses Bestätigungssignal kann mit den Signalen der Sensoreinheit S an die Steuerungseinheit 10 zur weiteren Displayansteuerung geleitet werden. Das Hinweisfeld 502 umfasst somit alle relevanten Hinweise, was beim Einlegen der Spritze zu beachten ist. In einer Variante der Erfindung umfasst das Hinweisfeld 502 auch Fehlerhinweise, die den Anwender auf mögliche Fehler bei der unmittelbar bevorstehenden Bedienung hinweisen. Das Hinweisfeld kann beispielsweise auch einen Warnhinweis umfassen, wenn die Sensoreinheit S detektiert hat, dass eine Heparinspritze 207 ohne eine Luer-Lock Verbindung verwendet werden soll. Der Hinweis kann beschreiben, dass sich die Verbindung zwischen Heparinspritze 207 und Schlauchsystem lösen kann und somit eine potentielle Gefahrenquelle darstellt.

Zum Einlegen und Anschließen der Heparinspritze 207 müssen einige Bedienschritte in einer bestimmten Abfolge an der Heparinpumpe 206 ausgeführt werden. Der Anwender wird erfindungsgemäß durch die Ausgabe von entsprechenden Hinweisen 502 auf dem Display D in entsprechender Reihenfolge unterstützt und geführt. Es können z.B. folgende Hinweise in Feld 502 mit einer jeweils dem Bauteil zugeordneten und entsprechenden Markierung in Feld 501 ausgegeben werden:
- Heparinspritze 207 an das arterielle Schlauchsystem anschließen.
- Griffstück 2064 durch Drücken der Klemmhebel 2065 in die untere Position bringen.
- Heparinspritze 207 zwischen die Spannhebel 2061 einlegen. Die Flügel 2071 des Spritzenzylinders müssen sich zwischen den Spannhebeln 2061 und dem Bügel 2062 befinden.
- Griffstück 2064 durch Drücken der Klemmhebel 2065 in die Startposition bringen. Der Stempel 2072 des Spritzenkolbens muss sich zwischen den Klammern 2063 des Griffstücks 2064 befinden.
- Ist der Stempel des Spritzenkolbens ohne Drücken der Klemmhebel nicht mehr verschiebbar, wurde die Heparinspritze 207 korrekt fixiert.
- Falls noch geschlossen, die Klemme der Heparinleitung öffnen.

Für den letzten Anwendungsfall ist es in einer vorteilhaften Ausführungsform der Erfindung vorgesehen, dass das Öffnen der Klemme für die Heparinleitung bzw. der Zustand der Klemme als Betriebsmittel 200 über einen entsprechenden Sensor erfasst wird, um daraus automatisch das zugeordnete Menü bzw. die jeweilige Bildseite 500 auf dem Display D zur Anzeige zu bringen.

Wird beispielsweise die Abdeckung 204 des extrakorporalen Blutbehandlungsmoduls während der Behandlung geöffnet, wird dies automatisch durch z.B. an den Türen bzw. Abdeckungen 204 angeordnete Sensoren der Sensoreinheit S erfasst und an die Steuerungseinheit 10 weitergeleitet. Dies deutet auf einen außerplanmäßigen Eingriff hin. Erfindungsgemäß können dem Anwender der vermutlichen Situation entsprechende Optionen zur Bedienung auf dem Display D aufgezeigt werden, wie z.B. zur Behandlungsunterbrechung und Hinweise zur Überführung des Gerätes 20 in einen sicheren Status. Dabei stoppt die Blutpumpe und der Patient wird an der venösen und arteriellen Zugangsstelle durch zu aktivierende Schlauchklemmen vom extrakorporalen Blutkreislaufmodul getrennt. Als vorteilhaft erweist es sich, dass das Gerät 20 auch in Notfällen sehr schnell, gezielt und einfach auch von einem weniger versierten Personal bedient werden kann, da eine schrittweise Anweisung der Bedienschritte situationsspezifisch ausgegeben werden kann. Diese Ausführung erhöht die Patientensicherheit.

Gemäß einer bevorzugten Ausführung der Erfindung werden die Bedienelemente und Schalter des Gerätes 20, die in einem Notfall zu bedienen sind, an prominenter Position hervorgehoben auf dem Display D dargestellt (z.B. durch eine vergrößerte oder blinkende Darstellung, eine andere Farbgebung oder durch entsprechende Animation).

Gemäß einer bevorzugten Ausführung der Erfindung bleibt die erzeugte Displayausgabe 500 solange eingeblendet bis der Anwender auf einem der Bestätigungsfelder 504 eine finale Abfrage am Ende einer Beschreibung bestätigt oder quittiert, dass eine andere Bedienung geplant ist und z.B. doch keine Heparinspritze verwendet werden soll. Daraufhin kann eine neue Bildseite 500 erzeugt werden, die den Anwender auffordert, die teileingelegte oder falsch eingelegte Spritze wieder zu entfernen. Auch dieser Eingriff wird über die Sensoreinheit S erfasst.

In einer vorteilhaften Weiterbildung kann das Verfahren zum Zwecke einer Überprüfung und/oder Korrektur unterbrochen werden. Dabei ist es vorgesehen, dass der Anwender die Spritze 207 beispielsweise zur Korrektur (nochmals) aus der Heparinpumpe 206 entnimmt und anschließend wieder einlegt. Dies wird über einen Sensor erkannt. Vorzugsweise wird auch der Zeitbezug mit erfasst, so dass die Sensoreinheit S auch erfasst, wann und wie oft ein Betriebsmittel 200, in diesem Fall die Heparinpumpe 206, bedient worden ist. Diese Signale können in der Steuerungseinheit 10 so verrechnet werden, dass die erzeugte Bildseite 500 mit Hinweisen zum Einlegen der Heparinspritze 207 weiterhin dargestellt wird. Beispielsweise wird nach dem Einlegen der Spritze ein sogenannter initialer Heparin-Ankoppeltest durchgeführt, bei dem ca. 0,5 ml Heparinlösung verabreicht werden. Vor Ausführung des Ankoppeltests muss der Anwender das verabreichte Heparinvolumen an der Spritze kontrollieren, da in diesem Fall das Fördervolumen von der oben genannten Menge abweichen kann. Dieser Kontrollschritt kann auf der erzeugten Bildseite 500 ausgegeben werden. Optional kann eine Erklärung ausgegeben werden, dass das geförderte Volumen gegebenenfalls bei der Berechnung der Heparingesamtmenge berücksichtigt werden muss. Während der Behandlung wird der Heparin-Ankoppeltest beim Einlegen oder beim Wechsel der Spritze und bei Wechsel des arteriellen Schlauchsystems automatisch wiederholt mit den zugeordneten Bildseiten 500 auf dem Display D.

Erfindungsgemäß werden die erzeugte Displayausgabe und der zugeordnete Zustand in einem Speicher gespeichert. Dies hat den Vorteil, dass die Ausgabe auch für andere Fälle direkt aus dem Speicher abgerufen werden kann, falls der jeweilige Zustand erneut eintritt. Kommt es in dem oben dargestellten Beispiel zu einem späteren Zeitpunkt - nach dem Entnehmen und dem Wiedereinsetzen der Spritze 207 in die Heparinpumpe 206 - zu einem Fehler, der ein erneutes Einlegen der Spritze notwendig macht, so kann zum Zwecke der Fehleranalyse vor dem oder bei dem erneuten Einlegen der Spritze die jeweilige Bildseite 500 angezeigt bleiben oder erneut angezeigt werden, falls zwischenzeitlich andere Bildseiten ausgegeben worden sind.

In einer vorteilhaften Weiterbildung kann eine erzeugte Bildseite 500 auch für die Darstellungen von anderen Menüs auf dem Display D abgelegt werden. Des Weiteren kann die Bildseite 500 und/oder die dafür erzeugten Darstellungen und/oder Felder 501, 502, 5021, 5022, 504 als vorbereitender Hinweis für den Anwender zur Verfügung gestellt und ausgegeben werden.

In einer anderen vorteilhaften Weiterbildung der Erfindung ist auf der erzeugten Bildseite 500 eine Korrekturtaste vorgesehen. Bei einer Betätigung der Korrekturtaste bleibt die erzeugte Bildseite 500 weiterhin dargestellt oder es wird auf die im jeweils vorhergehenden Schritt erzeugte Bildseite zurückgesprungen, um den Anwender bei dem jeweils vorhergehenden Schritt zu unterstützen, wenn dieser erneut und in korrigierter Form ausgeführt werden muss.

In einer anderen vorteilhaften Weiterbildung der Erfindung umfasst die Steuerungseinheit 10 zusätzlich ein Autorisierungsmodul, das dazu bestimmt ist, mit einem Näherungssensor in Datenaustausch zu stehen und die Autorisation einer sich dem Gerät 20 nähernden Bedienperson zu erfassen. Dazu können Identifikationseinheiten bereitgestellt werden, die die Bedienpersonen an sich tragen und zur eineindeutigen Identifikation der Person und zur Bestimmung von deren Autorisierung in Hinblick auf die Gerätebedienung dienen. Die Identifikationseinheiten können in andere elektronische Bauteile integriert sein (Mobilfunkgerät, Smartcard, Smartwatch, Uhr etc.). Die Daten der Identifikationseinheiten können z.B. über geeignete drahtlose Kommunikationskanäle übertragen werden, z.B.radiowellenbasierte Kanäle, wie NFC oder RFID, oder über Bluetooth oder anderen Protokollen zum drahtlosen Austausch von Daten. Die Steuerungseinheit 10 ist dann erfindungsgemäß dazu bestimmt, eine situationsabhängige und zustandsbasierte Ausgabe auf dem Display D zu erzeugen. Dabei fließt in den Zustand auch die Autorisation der Bedienperson ein, so dass nur solche Bedienschritte zur Anzeige gebracht werden, für die die sich jeweils nähernde Bedienperson auch autorisiert ist.

In einem Ausführungsbeispiel soll die Verwendung von Pumprotoren als Betriebsmittel 200 überwacht werden. Schlauchrollenpumpen der Dialysegerate 20 sind mit gefederten und in Radialrichtung beweglichen Rollen ausgestattet. Ersteres bewirkt, dass bei einer Blockade des Fluidwegs flussabwärts der Schlauchrollenpumpe ein unzulässig hoher Druck in der zu pumpenden Flüssigkeit (Blut) herrscht, was einerseits zu Blutschädigung durch Hämolyse führen kann, und andererseits zu potentiellen Schlauchrupturen in Folge von Überdruck. Durch die federnde Lagerung der Rolle wird die vollständige Okklusion des Schlauches durch die Rolle aufgehoben, wenn der Flüssigkeitsdruck den Anpressdruck der Rolle übersteigt und somit der maximale Flüssigkeitsdruck auf den durch die Federung bestimmten Anpressdruck begrenzt. Dieser kann je nach Behandlung differieren. Beispielsweise werden für pädiatrische Behandlungen spezielle Pumprotoren benutzt. Hierzu sind die Pumprotoren auswechselbar und können erfindungsgemäß über Identifizierungseinrichtungen verfügen, die z.B. als Bestandteil der Sensoreinheit S bereitgestellt werden können. Die Identifizierungseinrichtung dient dabei dazu, dass die Steuerungseinheit 10 automatisch erfassen kann, welcher Rotor verwendet wird (beispielsweise wie in der DE 10 2010 002 133 A1 beschrieben). Die radial beweglichen Rotorarme der Pumpe können beim Aufrüsten der Maschine bzw. des Gerätes 20 zusammengedrückt werden, um das Einlegen des Schlauches in das Pumpenbett zu erleichtern.

Erfindungsgemäß werden in einer ersten, retrospektiven Variante der Erfindung alle oder ausgewählte bereits ausgeführte Bedienungen am medizinischen Gerät 20 und an seinen Betriebsmitteln 200 über die Sensoreinheit S erfasst und zur zustandsabhängigen Displayausgabe verwendet. Im obigen Beispiel also sowohl für die Bedienung des Gerätes 20 zum Auswechseln des Rotors als auch für die Bedienung durch das Zusammendrücken der Rotorarme, wie vorstehend beschrieben. Dafür werden erfindungsgemäß geeignete Sensoren der Sensoreinheit S vorgehalten. Diese dienen zur Erfassung des Vorgangs am Gerät 20 bzw. der am Gerät und seiner Betriebsmittel 200 ausgeführten Bedienschritte. Die ausgeführten Bedienschritte werden in einem Zustand repräsentiert. Darauf basierend wird erfindungsgemäß eine zustandsbasierte Ausgabe auf dem Display D erzeugt.

Erfindungsgemäß werden in einer zweiten, prospektiven Variante der Erfindung die erfassten und bereits ausgeführten Bedienungen am medizinischen Gerät 20 und an seinen Betriebsmitteln 200 über die Sensoreinheit S verwendet, um daraus zukünftige, wahrscheinliche, weitere Bedienschritte zu berechnen und diese zur zustandsabhängigen Displayausgabe zu verwenden. Der Zustand umfasst dann nicht nur die bereits ausgeführten Bedienschritte, sondern auch zukünftige oder wahrscheinlich intendierte Bedienschritte. Dazu kann ein Speicher vorgesehen sein, auf den ein statistischer Algorithmus zur Berechnung zugreift und in dem Referenzdaten abgelegt sind. So kann beispielsweise zu einem erfassten Zustand immer ein Folge-Zustand als Referenz abgelegt werden, auf den der Algorithmus zugreifen kann. Wenn z.B. von der Sensoreinheit S bestimmte Sensorsignale erfasst werden, die einen Zustand definieren, der auf das Ausführen einer pädiatrischen Behandlung folgern lassen, so wird erfindungsgemäß automatisch eine Displayausgabe mit einem speziellen pädiatrischen Menü erzeugt. Wenn hingegen als Zustand "Schlaucheinlegen geplant" erfasst ist, dann wird erfindungsgemäß eine Step-by-Step-Anleitung zum Einlegen des Schlauches für die jeweilige Pumpe ausgegeben. Dabei kann vorzugsweise auch nach den jeweiligen Betriebsmitteln 200 differenziert werden (also z.B. differenziert für eine Blutpumpe im Single-Needle-Betrieb oder im Zweinadel-Betrieb oder für eine Substituatpumpe).

In einer Ausführungsform der Erfindung wird die Darstellung auf dem Display D somit durch die Sensordaten der Sensoreinheit S ausgelöst oder getriggert. Damit wird der technische Vorteil erreicht, dass Ausgabesignale erzeugt werden, die spezifisch auf den erfassten Zustand des Gerätes 20 mit seinen Betriebsmitteln 200 abgestimmt sind. Es ist nicht mehr notwendig, ein vorkonfiguriertes Menü zu durchlaufen, was zu einer deutlichen Zeitersparnis beiträgt.

In einem anderen Anwendungsbeispiel wird das erfindungsgemäße Verfahren auf eine Haltevorrichtung für einen Dialysefilter als Betriebsmittel 200 angewendet. Dazu sind Sensoren vorgesehen, die erkennen, ob ein Dialysator eingelegt ist. Je nachdem, ob das Eingelegtsein eines Dialysators und gegebenenfalls ein Dialysatortyp erkannt werden, wird eine spezifische Anzeige für die weiteren Bedienschritte angezeigt. Der Dialysatortyp kann z.B. durch Abfrage von Sensordaten ermittelt werden, die einen Öffnungswinkel einer Klemme des Gerätes 20 messen.

Zusammenfassend zielt die Erfindung darauf ab, dass auf Basis von automatisch erfassten Sensorsignalen ein Gerätezustand berechnet wird, der alle (oder nur die als relevant konfigurierten) Betriebsmittelzustände (z.B. Klappe 1 geöffnet, Klappe 2 geschlossen, Schalter 1 an, Schalter 2 aus, Abdeckung offen etc.) umfasst. Auf Basis des berechneten Zustandes wird eine Ausgabe für das Display erzeugt. Dies kann eine Bildseite 500 sein, die als Startseite für ein bestimmtes Betriebsmenü dient.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Es liegt somit z.B. ebenso im Rahmen der Erfindung neben der Heparinpumpe andere Betriebsmittel 200 des medizinischen Gerätes 20 auf deren korrekte Bedienung zu überprüfen. Dabei kann es sich z.B. um handbetätigte Betriebsmittel oder um automatische Elemente handeln. Für einen Fachmann ist es insbesondere offensichtlich, dass die Erfindung nicht nur für Dialysegeräte angewendet werden kann, sondern auch für andere medizinische Geräte, die über ein Display D bedient und gesteuert werden müssen.

Des Weiteren können die Bauteile des medizinischen Gerätes 20 zur zustandsabhängigen Steuerung des medizinischen Gerätes 20 über eine adaptiv erzeugte Displayausgabe auf mehrere physikalische Produkte verteilt realisiert werden.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHEN

- D: Display
- S: Sensoreinheit
- 10: Steuerungseinheit
- 100: Steuerlogik
- 1001: Treiber
- 1002: Controller
- 1003: Speicher, insbesondere Grafikspeicher

- 20: Medizinisches Gerät, insbesondere Dialysegerät
- 200: Betriebsmittel
- 202: extrakorporales Behandlungsmodul
- 204: Abdeckung, insbesondere Türe
- 206: Heparinpumpe
- 2061: Spannhebel
- 2062: Haltebügel
- 2063: Klammern
- 2064: Griffstück
- 2065: Klemmhebel
- 207: Heparinspritze
- 2071: Flügel des Spritzenzylinders
- 2072: Stempel des Spritzenkolbens
- 208: Blutpumpe
- 210: Substituatpumpe
- 212: Substituatport
- 214: Rinne
- 218: Bauteilgruppe, insbesondere zur venösen Überwachung

- 500: Bildseite, insbesondere Menüseite oder Startmenüseite
- 501: dreidimensionale Darstellung eines Betriebsmittels
- 502: Hinweisfeld
- 5021: Textfeld
- 5022: Markierfeld
- 504: Bestätigungsfeld

- a: Erfassen von Sensordaten
- b: Berechnen von Steuerbefehlen
- c: Zustandsabhängige Ansteuerung des Displays

## Patentansprüche

1. Medizinisches Gerät (20), das über ein Display (D) bedient und gesteuert wird, mit folgenden in Datenaustausch stehenden Modulen:
- einer Steuerungseinheit (10) zur Ansteuerung eines Displays (D) zum Betrieb des medizinischen Gerätes (20), die dazu bestimmt ist, aus erfassten Signalen einen Zustand des medizinischen Gerätes (20) zu ermitteln und mittels einer Steuerlogik (100) Steuerbefehle zur zustandsabhängigen Ansteuerung des Displays (D) zu berechnen, wobei je nach erfasstem Zustand unterschiedliche zustandsspezifische Menüs zur Darstellung auf einem Display kommen, um den Anwender beim Betrieb des Gerätes zu unterstützen und/oder ihn auf etwaige Fehler und/oder nächste Schritte hinzuweisen;
- Betriebsmitteln (200), die an das medizinische Gerät (20) angeschlossen sind und zum Betrieb des medizinischen Gerätes (20) bedient werden müssen;
- einem Display (D), auf dem zur Bedienung des medizinischen Gerätes (20) und/oder seiner Betriebsmittel (200) Displayausgaben aus einem Grafikspeicher (1003) ausgegeben werden und das zur Bedienung und Steuerung des medizinischen Gerätes (20) bestimmt ist, wobei der Grafikspeicher (1003) ausgebildet ist, Displayausgaben und ein der Displayausgabe zugeordneten Zustand zu speichern;
- einer Sensoreinheit (S), die am medizinischen Gerät (20) und ausgewählten Betriebsmitteln (200) angeordnet ist und die dazu bestimmt ist, automatisch einen Zustand des medizinischen Gerätes (20) und der jeweiligen Betriebsmittel (200) zu erfassen und zur Berechnung der Steuerbefehle an die Steuereinheit (10) weiterzuleiten und automatisch Signale zu erfassen und an die Steuerungseinheit (10) weiterzuleiten; und
wobei eine Displayausgabe für genau dasjenige Betriebsmittel ausgegeben wird, auf dem eine Zustandsveränderung erfasst worden ist.

2. Medizinisches Gerät (20) nach Anspruch 1, **dadurch gekennzeichnet, dass** das Betriebsmittel (200) eine Pumpe, insbesondere eine Heparinpumpe (206) zur Aufnahme einer Heparinspritze (207), eine Haltevorrichtung für einen Dialysefilter und/oder eine Aufnahmeeinrichtung für medizinische Einwegartikeln ist.

3. Medizinisches Gerät (20) nach einem der vorangehenden Ansprüche, **dadurch gekennzeichnet, dass** die Sensoreinheit (S) zumindest einen und vorzugsweise mehrere Sensoren an unterschiedlichen Bauteilen und/oder Positionen des medizinischen Gerätes (20) umfasst.

4. Medizinisches Gerät (20) nach einem der vorangehenden Ansprüche, **dadurch gekennzeichnet, dass** die Sensoreinheit (S) Sensoren umfasst, die an allen oder ausgewählten Betriebsmitteln (200) des medizinischen Gerätes (20) angeordnet sind und/oder die dazu ausgebildet sind, unterschiedliche physikalische oder chemische Messgrößen zu erfassen.

5. Medizinisches Gerät (20) nach einem der vorangehenden Ansprüche, **dadurch gekennzeichnet, dass** die Sensoreinheit (S) zumindest einen Sensor, Schalter, Taster und/oder einen Potentiometer umfasst.

6. Medizinisches Gerät (20) nach einem der vorangehenden Ansprüche, **dadurch gekennzeichnet, dass** die Steuerungseinheit (10) einen Sensortrigger-Schalter umfasst, über den sie aktivierbar und deaktivierbar ist.

7. Verfahren zur Ansteuerung eines Displays (D) eines medizinischen Gerätes (20), das über das Display (D) bedient und gesteuert wird, mit folgenden Verfahrensschritten:
- Erfassen (a) von Sensordaten, die einen Zustand des medizinischen Gerätes (20) mit seinen Betriebsmitteln (200) repräsentieren;
- Berechnen (b) von Steuerbefehlen zur zustandsabhängigen Ansteuerung (c) des Displays (D) in Abhängigkeit von den erfassten Sensordaten;
- Darstellen von unterschiedlichen zustandsspezifischen Menüs je nach erfasstem Zustand, um den Anwender beim Betrieb des Gerätes zu unterstützen und/oder ihn auf etwaige Fehler und/oder nächste Schritte hinzuweisen, wobei eine Ausgabe auf dem Display (D) für genau dasjenige Betriebsmittel (200) ausgegeben wird, auf dem eine Zustandsveränderung erfasst worden ist; und
- Speichern der Displayausgabe und einem der Displayausgabe zugeordnetem Zustand in einem Grafikspeicher (1003).

8. Verfahren nach Anspruch 7, bei dem der Zustand zumindest eine Zustandsgröße umfasst und wobei insbesondere jeweils eine Zustandsgröße einen Zustand jeweils eines Betriebsmittels (200) kennzeichnet.

9. Verfahren nach einem der Ansprüche 7 oder 8, bei dem die Bedienung eines jeweiligen Betriebsmittels (200) zum Betrieb des medizinischen Gerätes (20) eine Abfolge von Bedienschritten umfasst und dass jeweils ein Bedienschritt zumindest einer Displayausgabe zugeordnet ist.

10. Verfahren nach einem der Ansprüche 7 bis 9, bei dem die Ausgabe auf dem Display (D) eine interaktive Benutzerführung mit einer Abfolge von vorzugsweise hierarchisch strukturierten Betriebsmenüdarstellungen mit Untermenüdarstellungen fürjeweils ein Betriebsmittel (200) umfasst, wobei die Abfolge von Betriebsmenüdarstellungen in Abhängigkeit von den erfassten Sensordaten bestimmt wird.

11. Verfahren nach einem der Ansprüche 7 bis 10, bei dem die Ausgabe auf dem Display (D) zur interaktiven Benutzerführung während des Betriebs oder während eines Aufrüstens des medizinischen Gerätes (20) erfolgt, und wobei abhängig von den erfassten Sensordaten eine Betriebsmenüdarstellung für ein solches Betriebsmittel zur Anzeige ausgewählt wird, auf dem eine Zustandsveränderung erfasst worden ist.

12. Computerprogrammprodukt für ein medizinisches Gerät (20), das in einen internen Speicher einer digitalen Berechnungseinheit geladen werden kann und Softwareroutinen umfasst, mit denen die Schritte des Verfahrens gemäß den Ansprüchen 7 bis 11 ausgeführt werden, wenn die Softwareroutinen auf der digitalen Berechnungseinheit ausgeführt werden.

## Claims

1. Medical apparatus (20) operated and controlled via a display (D), with the following modules, being in data exchange:
- a control unit (10) for controlling a display (D) for operating the medical device (20), which is intended to determine a state of the medical device (20) from detected signals and to calculate control commands for the state-dependent controlling of the display (D) by means of a control logic (100), wherein, depending on the detected state, different state-specific menus are presented on a display in order to support the user in operating the device and/or to point out to him any errors and/or next steps;
- operating means (200) which are connected to the medical device (20) and have to be operated for the operation of the medical device (20);
- a display (D) on which display outputs are output from a graphics memory (1003) for operating the medical device (20) and/or its operating means (200) and which is intended for operating and controlling the medical device (20), the graphics memory (1003) being designed to store display outputs and a state associated with the display output;
- a sensor unit (S), which is arranged on the medical device (20) and on selected operating means (200) and which is intended to automatically detect a state of the medical device (20) and the respective operating means (200) and to forward them to the control unit (10) for calculation of the control commands and to automatically detect signals and forward them to the control unit (10); and
where a display output is shown for exactly that operating means (200) on which a change of state has been detected.

2. Medical apparatus (20) according to claim 1, **characterized in that** the operating means (200) is a pump, in particular a heparin pump (206) for receiving a heparin syringe (207), a holding device for a dialysis filter and/or a receiving device for disposable medical articles.

3. Medical apparatus (20) according to one of the preceding claims, **characterized in that** the sensor unit (S) comprises at least one and preferably several sensors on different components and/or positions of the medical device (20).

4. Medical apparatus (20) according to one of the preceding claims, **characterized in that** the sensor unit (S) comprises sensors which are arranged at all or selected operating means (200) of the medical device (20) and/or which are designed to detect different physical or chemical measured variables.

5. Medical apparatus (20) according to one of the preceding claims, **characterized in that** the sensor unit (S) comprises at least one sensor, switch, push button and/or potentiometer.

6. Medical apparatus (20) according to one of the preceding claims, **characterised in that** the control unit (10) comprises a sensor trigger switch, via which it can be activated and deactivated.

7. Method for controlling a display (D) of a medical device (20) which is operated and controlled via the display (D), comprising the following method steps:
- acquisition (a) of sensor data representing a state of the medical device (20) with its operating means (200);
- calculating (b) control commands for the state-dependent control (c) of the display (D) as a function of the acquired sensor data;
- displaying different state-specific menus depending on the detected state in order to support the user in operating the device and/or to inform him about possible errors and/or next steps, wherein an output is issued on the display (D) for exactly that operating operating means (200) on which a change of state has been detected; and
- Store the display output and a state assigned to the display output in a graphics memory (1003).

8. Method according to claim 7, in which the state comprises at least one state variable and wherein in particular one state variable in each case characterizes a state of a respective operating means (200).

9. Method according to one of claims 7 or 8, in which the operation of a respective operating means (200) for operating the medical device (20) comprises a sequence of operating steps and that in each case one operating step is assigned to at least one display output.

10. Method according to one of claims 7 to 9, in which the output on the display (D) comprises an interactive user guidance with a sequence of preferably hierarchically structured operating menu displays with submenu displays for a respective operating means (200), the sequence of operating menu displays being determined as a function of the sensor data detected.

11. Method according to one of claims 7 to 10, in which the output on the display (D) takes place for interactive user guidance during operation or during upgrading of the medical device (20), and wherein, depending on the sensor data detected, an operating menu representation for such an operating device on which a change in state has been detected is selected for display.

12. A computer program product for a medical device (20) which can be loaded into an internal memory of a digital processing unit and comprises software routines with which the steps of the method according to claims 7 to 11 are performed when the software routines are executed on the digital processing unit.

## Revendications

1. Dispositif médical (20) commandé et contrôlé par un écran (D), avec les modules suivants dans l'échange de données :
- une unité de commande (10) pour la commande d'un écran (D) pour le fonctionnement du dispositif médical (20), qui est destinée à déterminer un état du dispositif médical (20) à partir de signaux détectés et à calculer des ordres de commande pour la commande de l'écran (D) en fonction de l'état au moyen d'une logique de commande (100), dans laquelle, en fonction de l'état détecté, différents menus spécifiques à l'état sont présentés sur un écran afin d'aider l'utilisateur à faire fonctionner le dispositif et/ou de lui indiquer les erreurs éventuelles et/ou les étapes suivantes ;
- les moyens de fonctionnement (200) qui sont connectés au dispositif médical (20) et qui doivent être utilisés pour le fonctionnement du dispositif médical (20) ;
- un écran (D) sur lequel sont sorties des sorties d'affichage d'une mémoire graphique (1003) pour le fonctionnement du dispositif médical (20) et/ou de ses moyens de fonctionnement (200) et qui est destiné à faire fonctionner et à commander le dispositif médical (20), la mémoire graphique (1003) étant conçue pour stocker des sorties d'affichage et un état associé à la sortie d'affichage ;
- une unité de détection (S), qui est disposée sur le dispositif médical (20) et les moyens de fonctionnement (200) sélectionnés et qui est destinée à détecter automatiquement un état du dispositif médical (20) et des moyens de fonctionnement (200) respectifs et à les transmettre à l'unité de commande (10) pour le calcul des commandes de contrôle et à détecter automatiquement des signaux et à les transmettre à l'unité de commande (10) ; et
par lequel une sortie d'affichage est émise pour exactement cet moyen de fonctionnement sur lequel un changement d'état a été détecté.

2. Dispositif médical (20) selon la revendication 1, **caractérisé en ce que** le moyen de fonctionnement (200) est une pompe, en particulier une pompe à héparine (206) pour recevoir une seringue à héparine (207), un dispositif de maintien pour un filtre de dialyse et/ou un dispositif de réception pour des articles médicaux jetables.

3. Dispositif médical (20) selon l'une des revendications précédentes, **caractérisé en ce que** l'unité de capteurs (S) comprend au moins un et de préférence plusieurs capteurs sur différents composants et/ou positions de l'appareil médical (20).

4. Dispositif médical (20) selon l'une des revendications précédentes, **caractérisé en ce que** l'unité de capteurs (S) comprend des capteurs qui sont disposés sur tous les moyens de fonctionnement (200) du dispositif médical (20) ou sur certains d'entre eux et/ou qui sont conçus pour détecter différentes variables physiques ou chimiques mesurées.

5. Dispositif médical (20) selon l'une des revendications précédentes, **caractérisé en ce que** l'unité de détection (S) comprend au moins un capteur, un interrupteur, un bouton-poussoir et/ou un potentiomètre.

6. Dispositif médical (20) selon l'une des revendications précédentes, **caractérisé en ce que** l'unité de commande (10) comprend un interrupteur à capteur de déclenchement au moyen duquel elle peut être activée et désactivée.

7. Procédé de commande d'un écran (D) d'un dispositif médical (20) qui est commandé et contrôlé par l'écran (D), comprenant les étapes de procédé suivantes :
- l'acquisition (a) de données de capteurs qui représentent un état du dispositif médical (20) avec ses moyens de fonctionnement (200) ;
- calcul (b) des commandes de contrôle pour l'activation en fonction de l'état (c) de l'affichage (D) en fonction des données détectées par les capteurs ;
- représentant différents menus spécifiques à l'état en fonction de l'état détecté afin d'aider l'utilisateur à faire fonctionner l'appareil et/ou de lui indiquer les erreurs possibles et/ou les étapes suivantes, dans lequel une sortie est émise sur l'écran (D) pour exactement ce moyen de fonctionnement (200) sur lequel un changement d'état a été détecté ; et
- Stocker la sortie d'affichage et un état attribué à la sortie d'affichage dans une mémoire graphique (1003).

8. Procédé selon la revendication 7, dans lequel l'état comprend au moins une variable d'état et dans lequel en particulier une variable d'état caractérise dans chaque cas un état d'un moyen de fonctionnement respectif (200).

9. Procédé selon l'une des revendications 7 ou 8, dans lequel l'utilisation d'un moyen de fonctionnement (200) respectif pour faire fonctionner le dispositif médical (20) comprend une séquence d'étapes de commande et dans lequel à chaque fois une étape de commande est associée à au moins une sortie d'affichage.

10. Procédé selon l'une des revendications 7 à 9, dans lequel la sortie sur l'écran (D) comprend un guidage interactif de l'utilisateur avec une séquence d'affichages de menus de fonctionnement de préférence structurés de manière hiérarchique avec des affichages de sous-menus pour un moyen de fonctionnment (200) respectif, la séquence des affichages de menus de fonctionnement étant déterminée en fonction des données de capteur détectées.

11. Procédé selon l'une des revendications 7 à 10, dans lequel la sortie sur l'écran (D) a lieu pour le guidage interactif de l'utilisateur pendant le fonctionnement ou pendant la mise à niveau du dispositif médical (20), et dans lequel, en fonction des données de capteur détectées, une représentation de menu de fonctionnement pour un tel moyen de fonctionnement sur lequel un changement d'état a été détecté est sélectionnée pour l'affichage.

12. Produit de programme informatique pour un dispositif médical (20), qui peut être chargé dans une mémoire interne d'une unité de calcul numérique et comprend des routines logicielles avec lesquelles les étapes de la méthode selon les revendications 7 à 11 sont exécutées lorsque les routines logicielles sont exécutées sur l'unité de calcul numérique.
